# EUROPEAN PATENT SPECIFICATION

(11) **EP 0 471 135 B1**
(45) Date of publication and mention of the grant of the patent: **11.06.1997**
(21) Application number: 90870129.5
(22) Date of filing: 14.08.1990
(51) Int. Cl.: A61K 7/06

(54) **Composition for the promotion of hair growth**
Haarwuchsmittel
Composition pour favoriser la croissance des cheveux

(43) Date of publication of application: 19.02.1992
(73) Proprietor: Hallam, Kenneth M., Cockeysville, Maryland 21030 (US)
(72) Inventor: Hallam, Kenneth M., Cockeysville, Maryland 21030 (US); Robinson,, Howard N., M.D., Lutherville, Maryland 21093 (US)
(74) Representative: Colens, Alain

(56) References cited:
- EP-A- 0 158 090
- FR-A- 336 814
- FR-A- 1 439 833
- FR-A- 2 159 400
- GB-A- 2 176 104
- GB-A- 2 177 919

## Description

### Field of the Invention

The present invention is directed to compositions, medicaments and methods for promoting the activity of the hair follicles of a living organism and, in particular, to compositions, medicaments and methods for the treatment of the hair follicles of a human scalp to promote the growth of hair thereon.

### Background of the Invention

At birth the average human scalp has approximately 100,000 - 150,000 hair follicles. Initially, hair follicles normally exhibit fine lanugo hair shafts, which are commonly referred to as "baby hair". This lanugo hair "matures", eventually, into terminal hair shafts, which is the hair most often exhibited during adolescence and adulthood. Thereafter, this terminal hair may remain as such, or it may develop into non-terminal vellus hair, which is commonly referred to as a "thinning" of the hair and, in more advanced stages, baldness.

The precise mechanism that triggers the development of non-terminal vellus hair is not precisely understood or agreed upon. However, there have been numerous attempts to provide an effective safe prophylaxis to arrest development of non-terminal vellus hairs and even to stimulate the regeneration of these vellus hairs into terminal hair shafts. Unfortunately, none of these attempts have proven to be fully satisfactory.

Thus, it can be seen that there remains a need for compositions, medicaments and methods for administering the same, which are effective in stimulating the development of terminal hairs from non-terminal vellus hairs, are safe for use with a human host, and which are easy to administer.

It is known to use procaine (FR 1439833) or niacin (nicotinic acid, GB 2176 104). To the knowledge of the applicant, however, a combination of both active ingredients to promote fresh hair growth (and/or retard hair loss) has never been proposed. It has now been found that surprising advantage may be observed with this particular combination.

### Summary of the Invention

Accordingly, it is a primary object of the present invention to alleviate the disadvantages and deficiencies of the prior art by providing compositions and medicaments that are effective for stimulating the development (growth) of terminal hairs from the hair bulb of non-terminal vellus hair follicles.

It is another primary object of the present invention to provide compositions and medicaments which may safely be used with a human host.

It is still another primary object of the present invention to provide methods for stimulating the development (growth) of terminal hairs from the hair bulb of non-terminal vellus hair follicles.

It is still yet another primary object of the present invention to provide methods of safely administering the compositions and medicaments to a human host.

It is a further object of the present invention to provide such compositions, medicaments and methods for the administration thereof, which are easy to perform.

In accordance with the teachings of the present invention, a topical composition for stimulating the growth of a hair bulb of a vellus hair follicle of a scalp is disclosed which composition comprises a combination of procaine hydrochloride and niacin in a pharmaceutically-acceptable carrier containing propylene glycol for penetrating the scalp and for carrying the niacin and procaine hydrochloride therewith.

Preferably, the said carrier includes a hydrophilic carrier that may be chosen from lanolin, butyl alcohol, absolute alcohol, isopropyl alcohol and dimethyl sulfoxide. Alternatively, a combination of two or more of these carriers may be employed. topically applying the composition to a scalp having a vellus hair follicle in need thereof.

### Brief Description of the Drawings

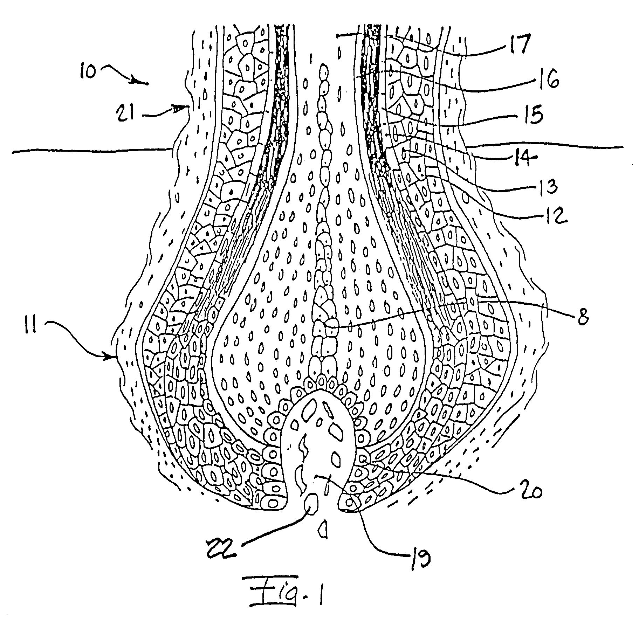

Figure 1 illustrates, in cross-section, a healthy terminal hair follicle.

### Description of the Preferred Embodiments

With reference now to Figure 1, the hair follicle 10 includes a hair bulb 11 at the lower end thereof. The hair bulb 11 of the average mature hair follicle 10 lies approximately 0.5 - 4 mm below the stratum corneum of the epidermus.

Generally, and as referred to herein, the hair follicle is comprised of seven layers: the outer root sheath 12; Henle's layer 13; Huxley's layer 14; cuticle of inner root sheath 15; the cuticle of hair shaft 16; the cortex of hair shaft 17; and the medulla of hair shaft 18. Further as referred to herein, the base of the hair follicle comprises a hair bulb 11 which surrounds a dermal papilla 19. Above the dermal papilla 19 is a germative cellular layer 20 which is the active growth portion of the hair shaft 21.

It is believed that the dermal papilla 19 receives nutrients and oxygen, etc. from the vascular superficial plexus 22 (which, as used herein, includes the superficial plexus and capillaries thereof) and carries these nutrients and oxygen to the growing portion 20 of the hair bulb 11. While not precisely understood or agreed upon, it is believed that growth of the hair shaft 21 (and hence, scalp hair) is a consequence of this interaction. It is our further belief that the presence of various androgens, such as testosterone, including Dihydrotestosterone, can in certain genetically predisposed individuals, result in the development of non-terminal vellus hairs.

Also, while not precisely understood or agreed upon, the presence of these androgens is believed to be related to the presence in the hair bulb of various receptors which are sensitive therefor (which may or may not be genetically predetermined) and/or the presence of active androgens and their metabolities.

The causes of hair loss which are thought to result from these factors include both scarring and non-scarring alopecia, specifically male and female pattern baldness, alopecia areata encompassing alopecia totalis and alopecia universalis. Additional non-scarring causes of alopecia which are thought to result from these factors include telogen and anagen effluvium. The scarring or cicatricial alopecias to be treated includes lichenplanopilaris, sarcoidosis, lupus erythematosus and other autoimmune diseases.

According to the teachings of the present invention, by increasing the vascular flow of oxygen and nutrients through the vascular plexus 22, the deleterious effect of the androgens can be superceded. This, it is believed, provides essential oxygen and nutrients to the cells of the germative cellular layer 20 and stimulates growth thereof resulting in the production of a terminal hair shaft 21 even in follicles 10 that had previously been vellus for numerous years.

The compositions and medicaments (therapeutic compounds) of the present invention utilize a powerful vasodilator, especially one having strong local effects, as an active ingredient. Also, a pharmaceutically-acceptable hydrophilic carrier is utilized as an adjunct. The active ingredients are niacin and procaine hydrochloride.

The pharmaceutically-acceptable carrier of the compositions and medicaments of the present invention contains propylene glycol. Other carriers such as lanolin, butyl alcohol, absolute alcohol (99% ethyl alcohol), isopropyl alcohol, and dimethyl sulfoxide (DMSO) may be employed in combination therewith. Indeed, the preferred compositions and medicaments are comprised of from 50% to 95% of propylene glycol and 15% to 30% of absolute alcohol. Other hydrophilic solutions, ointments, creams or gels may also be employed.

The amount of niacin of the compositions and medicaments described above may range from 0.01 - 4% of the total composition or medicament, with 0.1% of niacin being the preferred quantity, the amount of procaine hydrochloride ranges from 0.1 to 5% of the total composition.

These compositions and medicaments are topically applied directly to a scalp in need thereof by use of a dropper, a porous applicator, a roll top, small brush or even by massaging in with the hands or any other means which is suitable for topical application which are well known to those skilled in the art. When a porous applicator is used, a microporous applicator is preferred.

The precise amount of the compositions and medicaments to be applied is within the skill of the art to determine. However, the amount applied would be that quantity necessary to thinly saturate the affected area of the skin. In this regard, the application of from 1/6 to 1/3 cm³ of the compositions or medicaments will be preferable. These topical applications are preferably performed one to two times daily. The frequency of these applications may be increased or decreased, as needed, as would be obvious to one skilled in the art.

The protocol set forth above (and those below) will be substantially the same regardless of the type of alopecia being treated. Exact protocols are contemplated to vary from patient to patient depending on various factors, such as the medical health of the patient. Exact protocals to follow are well within the skill of the art to determine.

If desired, the compositions and medicaments may be incorporated in a shampoo or a gel form. In a shampoo, the compositions and medicaments include water, lauryl sulfate, a surfactant and, if desired, a fragrance. The shampoo is shaken prior to use to effect thorough mixing thereof and is hand-massaged into the scalp.

The invention will be more clearly perceived and better understood from the following specific examples.

[In each example, the proportions presented for the active ingredients, such as niacin or procaine HCl, are on a weight per unit volume basis (i.e. 5% procaine hydrochloride is 5 grams of procaine HCl per 100 cm³ of a pharmaceutically-acceptable topical carrier) and the proportions presented for the carrier consist of a percent by volume (i.e. 80% propylene glycol is 80 cm³ of propylene glycol per 100 cm³ of a pharmaceutically-acceptable topical carrier).]

### Example One

A 2% solution of procaine hydrochloride and 0.1% niacin in propylene glycol carrier is prepared. Approximately 1/6 to 1/3 cm³ of this composition or medicament is then placed in an eyedropper. The composition or medicament is then administered in a dropwise fashion on a portion of a human scalp in need thereof. Initially, the applications are made once every twelve hours. Eventually, the frequency of these applications may thereafter be decreased (or increased) as needed.

### Example Two

A 2% solution of procaine hydrochloride and 0.1% niacin in a propylene glycol carrier is prepared. Approximately 1/6 to 1/3 cm³ of this composition or medicament is placed in a container having a microporous applicator. This composition or medicament is then administered by application through the microporous applicator, so as to thinly saturate the afflicted area of the human scalp in need thereof. Initially, these applications are made every twelve hours. Eventually, the frequency of these applications may thereafter be decreased (or increased) as needed.

## Claims

1. A topical composition for stimulating growth of a hair bulb of a vellus hair follicle of a scalp, the composition being characterised in that it comprises a combination of niacin and procaine hydrochloride in a pharmaceutically acceptable carrier containing propylene glycol for penetrating the scalp and for carrying the niacin and procaine hydrochloride therewith.

2. The composition of claim 1 wherein the amount of niacin ranges from 0,01 to 4 % of the total composition, and the amount of procaine hydrochloride ranges from 0.1 to 5% of the total composition.

3. The composition of claim 1 or 2 wherein the pharmaceutically acceptable carrier includes a carrier chosen among lanolin, butyl alcohol, absolute alcohol, isopropyl alcohol and dimethyl sulfoxide, or a combination of two or more of these carriers.

## Patentansprüche

1. Topische Zusammensetzung zum Stimulieren des Wachstums einer Haarzwiebel eines Vellus-Haarfollikels der Kopfhaut, wobei die Zusammensetzung dadurch gekennzeichnet ist, daß sie eine Kombination aus Niacin und Procainhydrochlorid in einem pharmazeutisch verträglichen Träger aufweist, der zum Durchdringen der Kopfhaut und zum Transportieren von Niacin und Procainhydrochlorid Propylenglykol enthält.

2. Zusammensetzung nach Anspruch 1, wobei die Menge von Niacin im Bereich von 0,01 bis 4 % der gesamten Zusammensetzung und die Menge des Procainhydrochlorids im Bereich von 0,1 bis 5 % der gesamten Zusammensetzung liegt.

3. Zusammensetzung nach Anspruch 1 oder 2, wobei der pharmazeutisch verträgliche Träger einen Träger enthält, der ausgewählt ist aus Lanolin, Butylalkohol, absolutem Alkohol, Isopropylalkohol und Dimethylsulfoxid oder einer Kombination von zwei oder mehreren dieser Träger.

## Revendications

1. Composition topique pour stimuler la croissance du bulbe pileux d'un follicule de poil du cuir chevelu, ladite composition étant caractérisée en ce qu'elle comprend une combinaison de niacine et de chlorhydrate de procaine dans un excipient pharmaceutique acceptable contenant du propylene glycol pour la pénétration dans le cuir chevelu et pour y entraîner la niacine et le chlorhydrate de procaine.

2. Composition selon la revendication 1 dans laquelle la quantité de niacine est comprise entre 0,01 à 4 % de la composition totale, et la quantité de chlorhydrate de procaine s'étend de 0,1 à 5 % de la composition totale.

3. Composition selon la revendication 1 ou 2 dans laquelle l'excipient pharmaceutiquement acceptable comprend un excipient choisis parmi la lanoline, l'alcool butylique, l'alcool absolu, l'alcool isopropylique et le diméthylsulfoxide, ou une combinaison de deux ou plus de ces excipients.
